# EUROPÄISCHE PATENTSCHRIFT

(11) **EP 1 885 732 B1**
(45) Veröffentlichungstag und Bekanntmachung des Hinweises auf die Patenterteilung: **22.07.2009**
(21) Anmeldenummer: 06753212.7
(22) Anmeldetag: 30.05.2006
(51) Int. Cl.: C07F 15/00, C07F 9/6574, C07F 9/6571, C07F 9/6564, C07B 53/00, B01J 31/12

(54) **CHIRALE DIPHOSPHONITE ALS LIGANDEN IN DER RUTHENIUM-KATALYSIERTEN ENANTIOSELEKTIVEN REDUKTION VON KETONEN, ß-KETOESTERN UND KETIMINEN**
CHIRAL DIPHOSPHONITES AS LIGANDS IN THE RUTHENIUM-CATALYSED ENANTIOSELECTIVE REDUCTION OF KETONES ß-KETO-ESTERS AND KETIMINES
DIPHOSPHONITES CHIRAUX UTILISES EN TANT QUE LIGANDS DANS LA REDUCTION ENANTIOSELECTIVE DE CETONES, DE ß-CETO-ESTERS, DE ET CETIMINES, CATALYSEE PAR DU RUTHENIUM

(30) Priorität: 02.06.2005 DE 102005025797
(43) Veröffentlichungstag der Anmeldung: 13.02.2008
(73) Patentinhaber: Studiengesellschaft Kohle mbH, 45470 Mülheim an der Ruhr (DE)
(72) Erfinder: REETZ, Manfred, Theodor, 45470 Mülheim an der Ruhr (DE); LI, Xiaoguang, 45468 Mülheim an der Ruhr (DE)
(86) Internationale Anmeldenummer: PCT/DE2006/000929
(87) Internationale Veröffentlichungsnummer: WO 2006/128434

(56) Entgegenhaltungen:
- EP-A- 1 354 886
- WO-A-00/14096
- WO-A-01/07156
- WO-A-93/03839
- WO-A-02/057278
- WO-A2-02/22261
- WO-A2-03/018192
- WO-A2-20/04094442
- WO-A2-20/05047299
- FANG, X. ET AL.: "Synthesis and Structure of a Diphosphonite- Bridged Ruthenium Dication, {[(p-cymene)Ru(u-CI)2(u-1,2-di(2'2'-diethy l1',3'-propandioxy).phosphinoethane)]}2+" ORGANOMETALLICS, Bd. 22, 2003, Seiten 605-608, XP009076579
- STEINMETZ, BERNH. ET AL.: "Synthesis and Oxidation of Ruthenium allyl thioether-complexes ...." ZEITSCHRIFT FÜR NATURFORSCHUNG, Bd. 54b, 1999, Seiten 1265-1271, XP009076586
- REETZ ET AL: "Diphosphonites as highly efficient ligands for enantioselective rhodium-catalyzed hydrogenation" CHEMICAL COMMUNICATIONS - CHEMCOM, ROYAL SOCIETY OF CHEMISTRY, GB, Nr. 19, 1998, Seiten 2077-2078, XP002121420 ISSN: 1359-7345
- GOERTZ W ET AL: "Asymmetric Nickel-Catalysed Hydrocyanation of Vinylarenes by Applying Homochiral Xantphos Ligands" CHEMISTRY - A EUROPEAN JOURNAL, VCH PUBLISHERS, US, Bd. 7, Nr. 8, 2001, Seiten 1614-1618, XP002203116 ISSN: 0947-6539
- VLUGT,JARL IVAR,ET.AL: "Coordination Chemistry and Asymmetric Catalysis with a Chiral Diphosphonite" EUR. J. INORG. CHEM, 2004, Seiten 4193-4201, XP009076578
- KIM ET AL: "Novel ambidentate and chelate complexes of ruthenium with methylenebis(1,3,2-dithiaphospholane) and (methylimino)bis(1,3,2- dithiaphospholane) as ligands" INORGANIC CHEMISTRY, AMERICAN CHEMICAL SOCIETY, EASTON, US, Bd. 29, Nr. 19, 1990, Seiten 3896-3898, XP002415554 ISSN: 0020-1669
- DATABASE CA [Online] CHEMICAL ABSTRACTS SERVICE, COLUMBUS, OHIO, US; VIOTTE, M. ET AL: "New potentially cytotoxic thiolatogold(I) complexes of 1,1'-bis(diphenylphosphino)ferrocene" XP002430377 gefunden im STN Database accession no. 1996:726998 & INORGANICA CHIMICA ACTA , 253(1), 71-76 CODEN: ICHAA3; ISSN: 0020-1693, 1996,
- DATABASE BEILSTEIN [Online] BEILSTEIN INSTITUTE FOR ORGANIC CHEMISTRY, FRANKFURT-MAIN, DE; XP002430378 gefunden im XFIRE Database accession no. BRN 6253814 & DIEMERT ET AL.: PHOSPHORUS, SULFUR, Bd. 15, 1983, Seiten 155-164,

## Beschreibung

Die vorliegende Erfindung betrifft ein Verfahren zur Ruthenium-katalyslerten asymmetrischen Reduktion von Ketonen, β-Ketoestern und Ketiminen, wobei die Produkte enantiomerenreine oder angereicherte Alkohole bzw. Amine sind, welche industriell wertvolle Bausteine bei der Herstellung von Verbindungen wie Pharmazeutika, Pflanzenschutzmittel, Duftstoffe und natürliche Produkte oder Zwischenprodukte in deren Synthesen darstellen.

Die Übergangsmetall-katalysierte enantioselektive Reduktion von prochiralen Ketonen I, β-Ketoestern III und Ketimine V erfordern enatiomerenreine oder angereicherte chirale Alkohole II, β-Hydroxyester IV bzw. Amine VI, welche wertvolle Zwischenprodukte für die industrielle Herstellung einer Vielzahl von pharmazeutischen Wirkstoffen, Pflanzenschutzmitteln, Duftstoffen oder anderen Produkten sind (R. Noyori, Angew. Chem. Int. Ed. 2002, 41, 2008-2022; H.-U. Biaser, C. Malan, B. Pugin, F. Spindler, H. Steiner, M. Studer, Adv. Synth. Catal. 2003, 345, 103-151; M. J. Palmer, M. Wills, Tetrahedron: Asymmetry 1999, 10, 2045-2061). Eine Vielzahl von Katalysatorsystemen wurde für derartige Reduktionen entwickelt, entweder durch H₂-Hydrierung oder durch Transfer-Hydrierung z.B. unter Verwendung von Isopropanol als Wasserstoffdonor. Einige wenige bekannte chirale Liganden ergeben hohe Enantioselektivitäten (ee > 90 %) für einige aber nicht für alle Substrate (R. Noyori, Angew. Chem. Int. Ed. 2002, 41, 2008-2022; H.-U. Blaser, C. Malan, B. Pugin, F. Spindler, H. Steiner, M. Studer, Adv. Synth. Catal. 2003, 345, 103-151; M. J. Palmer, M. Wills, Tetrahedron: Asymmetry 1999, 10, 2045-2061).

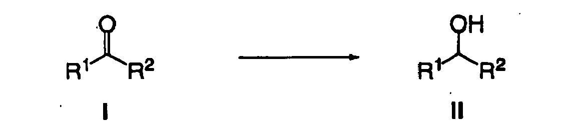

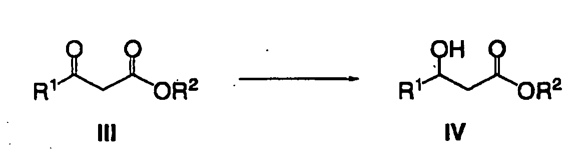

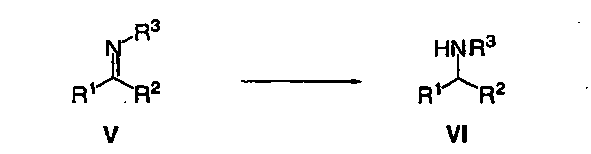

Nachteile der gängigen Methoden sind die hohen Kosten einer mehrstufigen Herstellung für die chiralen Liganden, die zur Erlangung eines hohen ee-Wertes erforderlich sind, und die nur begrenzte allgemeine Anwendbarkeit, z.B. viele interessante Ketone führen zu Alkoholen mit geringen Enantioselektivitäten. Beispielsweise enthält der Ru-Katalysator mit optisch aktivem BINAP und einem chiralen Diamin als zwei teure Liganden (R. Noyori, Angew. Chem. Int. Ed. 2002, 41, 2008-2022). In einem der derzeit effektivsten Verfahren zur asymmetrischen Ketonreduzierung, welches auch von Noyori beschrieben wurde, wird Ru(II), das durch eine aromatische Verbindung und einem monotosylierten chiralen Diamin komplexiert ist, verwendet, wobei die Komplexe als Katalysatoren bei der Transfer-Hydrierung mit Isopropanol als Wasserstoffdonor unter basischen Bedingungen agieren (R. Noyori, S. Hashiguchi, Acc. Chem. Res. 1997, 30, 97-102). Die Nachteile dieses Katalysatorsystems liegen in der mühsamen Herstellung der chiralen tosylierten Diaminliganden und der Tatsache, dass im allgemeinen nur Aryl-Alkylketone (I mit R¹ = Aryl und R² = Alkyl) mit hoher Enantioselektivität (ee > 90 %) reagieren, während viele Alkyl-Alkylketone (I mit R¹ = Alkyl und R² = ein davon unterschiedliches Alkyl) zu nur mäßigen oder geringen Enantioselektivitäten führen. Beispielsweise reduziert der beste Noyorl-Katalysator Methylcyclohexylketon (I, R¹ = CH₃; R² = c-C₆H₁₁) mit einem ee von nur 60 % (J. Takehara, S. Hashiguchl, A. Fujii, S.-I. Inoue, T. Ikariya, R. Noyori, Chem. Commun. (Cambridge, U. K.) 1996, 233-234). Dieses Katalysatorsystem wurde hinsichtlich der Enantioselektivität unter Verwendung von entsprechenden Ru(II)-Komplexen, welche der aromatische Ligand und der chirale tosylierte Diaminligand kovalent durch einen Ether miteinander verbunden sind, verbessert, was aber die Synthese des Ligandensystems weiter aufwendiger und teurer macht (A. M. Hayes, D. J. Morris, G. J. Clarkson, M. Wills, J. Am. Chem. Soc. 2005, 127, 7318-7319). Darüber hinaus wird selbst die Enantioselektivität für solche Alkyl-Alkylketone, wie Methylcyclohexylketon (I, R¹ = CH₃; R² = c-C₆H₁₁) nur geringfügig verbessert (ee = 69 %) (A. M. Hayes, D. J. Morris, G. J. Clarkson, M. Wills, J. Am. Chem. Soc. 2005, 127, 7318-7319).

In der WO 02/22261 wird ein Verfahren zur Hydroformulierung ethylenisch ungesättigter Verbindungen offenbart. Als Katalysator wird für die Reaktion eine Metallkomplexverbindung von Metallen der VIII. Nebengruppe mit mindestens einer Phosphor-, Arsen- oder Antimonhaltigen Verbindung als Liganden eingesetzt. Bei der Hydroformulierung von α-Oliphinen soll ein möglichst hoher Anteil an α-Aldehyden bzw. α-Alkoholen erzielt werden. Als Übergangsmetallkatalysatoren werden Rhodiumkomplexe eingesetzt.

Die vorliegende Erfindung beseitigt viele der oben beschriebenen Nachteile.

Gegenstand ist ein Verfahren zur asymmetrischen Ruthenium-katalysierten Reduktion von prochiralen Ketonen, β-Ketoestern und Ketiminen, worin chirale Rutheniumkomplexe hergestelit durch die Reaktion eines Rutheniumsalzes mit einem chiralen Diphosphonit, in welchem das Diphosphonit abgeleitet ist aus einem chiralen Diol mit der allgemeinen Struktur, gezeigt in Schema 1, verwendet werden 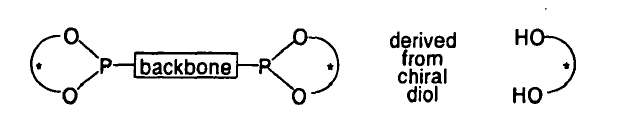 Scheme 1. Chiral phosphonites derived from chiral diols
das dadurch gekennzeichnet ist, dass Rückgrat achiral ist und sich von einem der Reste des **U1** - **U15** ableitet

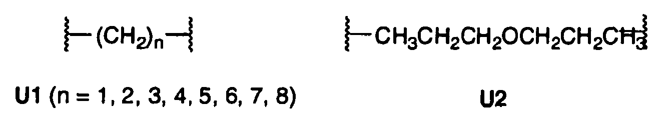

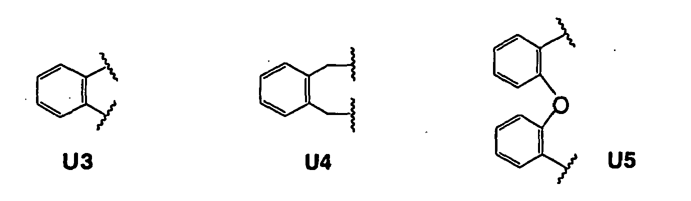

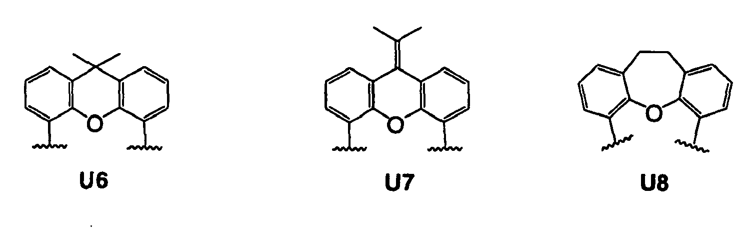

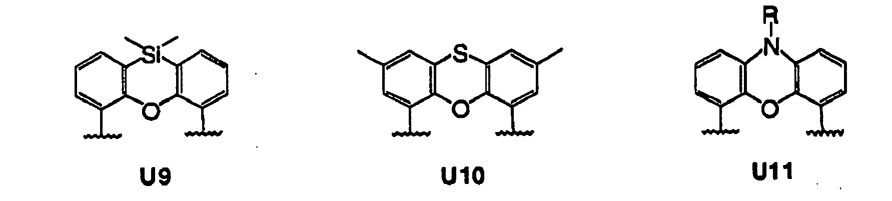

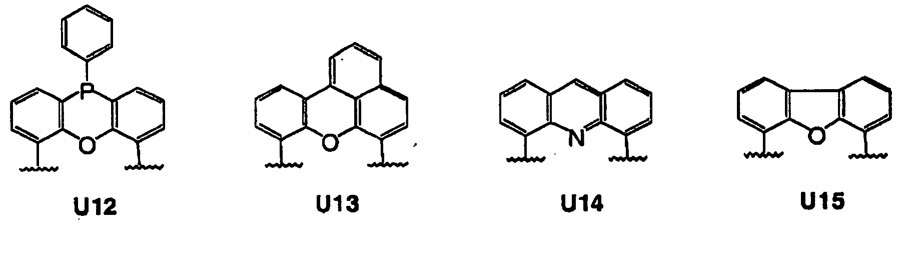

Die Erfindung nutzt Ruthenium-Komplexe mit preiswerten und auf einfache Weise erhältlichen chiralen Diphosphoniten. Phosphonite sind Verbindungen mit einer Kohlenstoff-Phosphor-Bindung und zwei Phosphor-Sauerstoff-Bindungen. Stickstoff-Analoga, d.h. Derivate der Phosphonite, in welchen ein oder beide Sauerstoff-Reste durch eine Aminogruppe ersetzt wurden, werden von der vorliegenden Erfindung ebenfalls umfasst. Die Liganden der vorliegenden Erfindung bestehen aus einem achiralen oder chiralen Rückgrat, an welches zwei Phosphonitreste gebunden sind, wobei jeder Rest einen chiralen Liganden, wie ein chirales Diol (Schema 1), enthält, wobei auch alle stereoisomeren Formen Bestandteil der Erfindung sind.

Viele dieser Diphosphonite und deren Stickstoff-Analoga wurden bereits in der Literatur beschrieben (M. T. Reetz, A. Gosberg, R. Goddard, S.-H. Kyung, Chem. Commun. (Cambridge, U. K.) 1998, 2077-2078; I. E. Nifant'ev, L. F. Manzhukova, M. Y. Antipin, Y. T. Struchkov, E. E. Nifant'ev, Zh. Obshch. Khim. 1995, 65, 756-760; J. I. van der Vlugt, J. M. J. Paulusse, E. J. Zijp, J. A. Tijmensen, A. M. Mills, A. L. Spek, C. Claver, D. Vogt, Eur. J. I-norg. Chem. 2004, 4193-4201; M. T. Reetz, A. Gosberg, Int. Pat. Appl. WO 00/14096, 2000), aber keine der beschriebenen Verbindungen wurde als Ligand in Ru(II)-katalysierten Reaktionen verwendet. Die vorliegende Erfindung umfasst auch die Herstellung dieser neuen Ru(II)-Komplexe und ihre Verwendung als Katalysatoren bei der asymmetrischen Reduzlerung von Ketonen; β-Ketoestern und Iminen.

Es ist bekannt, dass die Art des Rückgrates bei den Diphosphoniten beträchtlich variieren kann, was eine strukturelle Vielfalt bei der Herstellung der entsprechenden Ru(II)-Komplexe ermöglicht. Einfache Alkyl- oder substituierte Alkylketten, d.h. -(CH₂)-ₙ mit n = 1, 2, 3, 4, 5, 6, 7 oder 8, kann als Rückgrat dienen oder Alkylketten, die Heteroatome in der Kette enthalten, z.B. -CH₂CH₂CH₂OCH₂CH₂CH₂-, aber auch aromatische Reste wie o,o-disubstituierte Benzolderivate. Ein Beispiel für ein chirales Rückgrat ist das trans-1,2-disubstituierte Cyclopentanderivat. Als Rückgrat ausgeschlossen sind Ferrocenderivate, die einen Phosphorrest an jeder Cyclopentadienylgruppe aufweisen (I. E. Nifant'ev, L. F. Manzhukova, M. Y. Antipin, Y. T. Struchkov, E. E. Nifant'ev, Zh. Obshch. Khim. 1995, 65, 756-760; M. T. Reetz, A. Gosberg, R. Goddard, S.-H. Kyung, Chem. Commun. (Cambridge, U. K.) 1998, 2077-2078; M. T. Reetz, A. Gosberg, Int. Pat. Appl. WO 00/14096, 2000). Ein besonders preiswertes chirales Hilfsmittel am Phosphor in den Diphosphoniten sind neben vielen weiteren Möglichkeiten (R)- oder (S)-Dinaphthol (BINOL). Typische Beispiele sind im Folgenden dargestellt (VII-X):

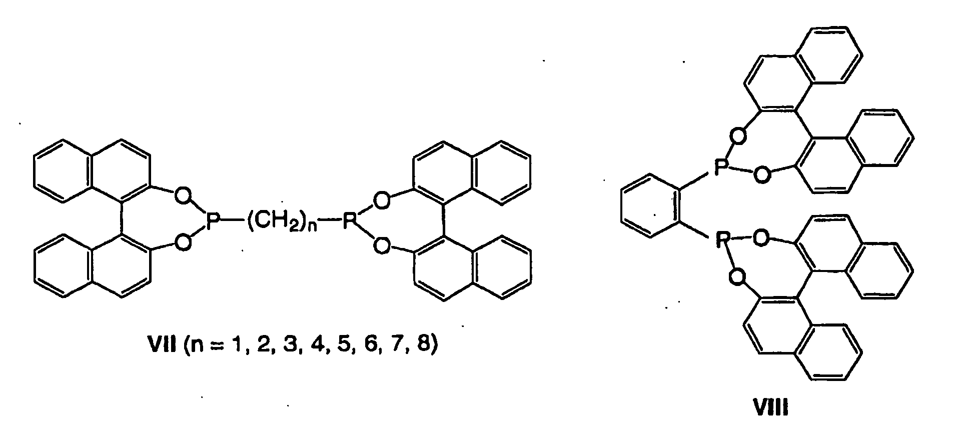

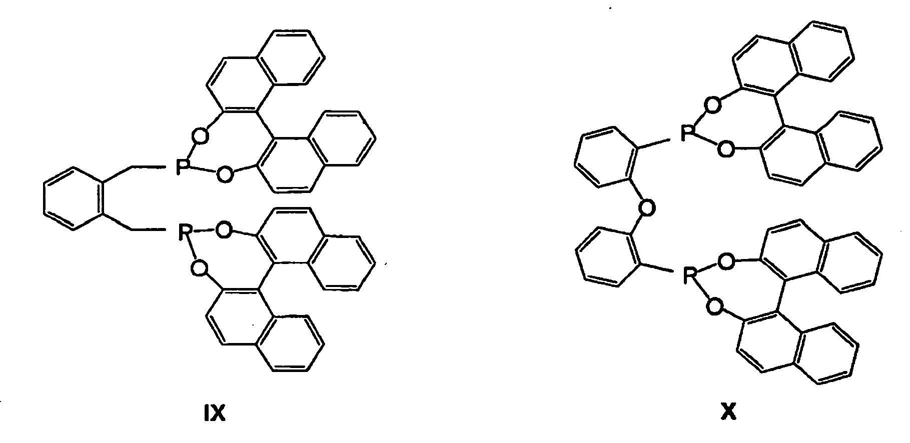

Des weiteren können typische Beispiele auch aus den Derivaten von Xanthen (z.B. XI oder XII), Homo-Xanthen (z.B. XIII), Sexanthen (z.B. XIV), Thixanthen (z.B. XV), Nixanthen (z.B. XVI), Phosxanthen (z.B. XVII), Benzoxanthen (z.B. XVIII), Acridin (z.B. XIX) oder Dibenzofuran (z.B. XX) hergestellt werden:

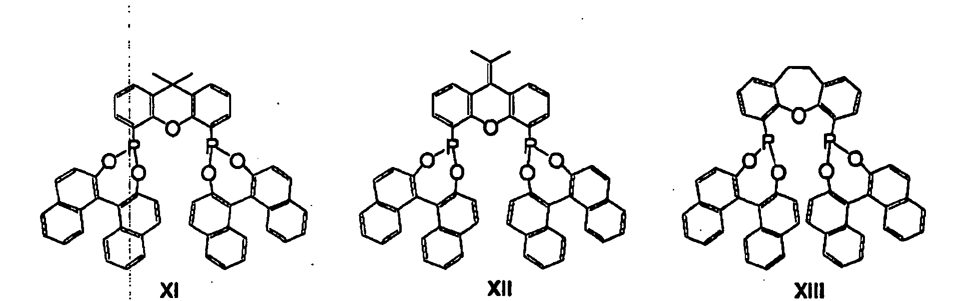

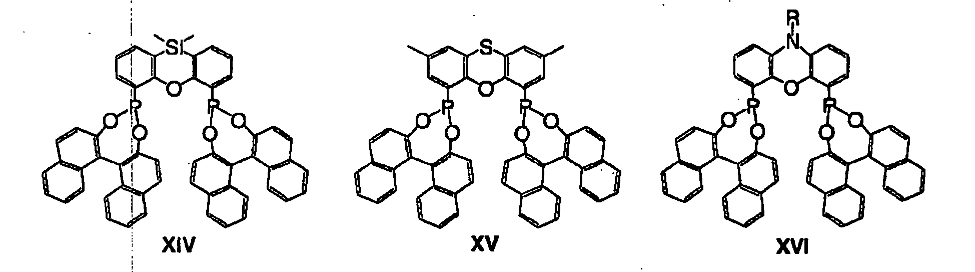

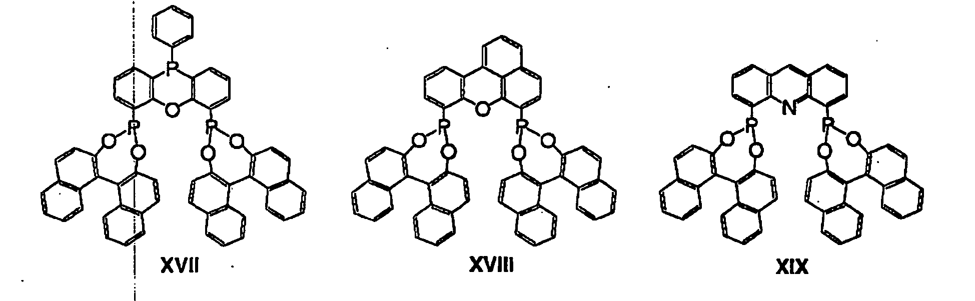

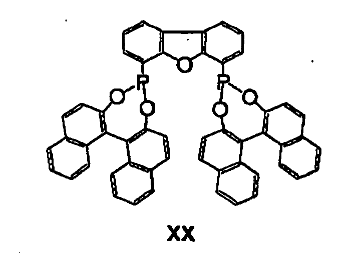

Auch wenn in allen oben beschriebenen Diphosphoniten das chirale Hilfsmittel am Phosphor BINOL (A) ist, ist die Erfindung nicht auf dieses spezielle chlrale Diol beschränkt. Octahydro-BINOL (B) kann neben vielen anderen auch verwendet werden.

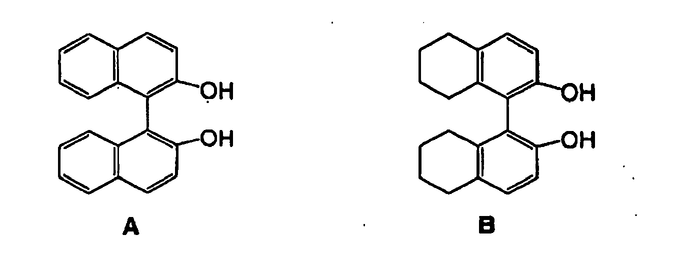

Andere axial chirale Diole können ebenfalls verwendet werden, viele von ihnen wurden gemäß Literatur unter Verwendung von effizienten Syntheseprozessen hergestellt, z.B. substituierte BINOL-Derivate C, substituierte Diphinol-Derivate D mit axialer Chiralität und Diole mit axialer Chiralität, die die Heterocyclen gemäß E enthalten.

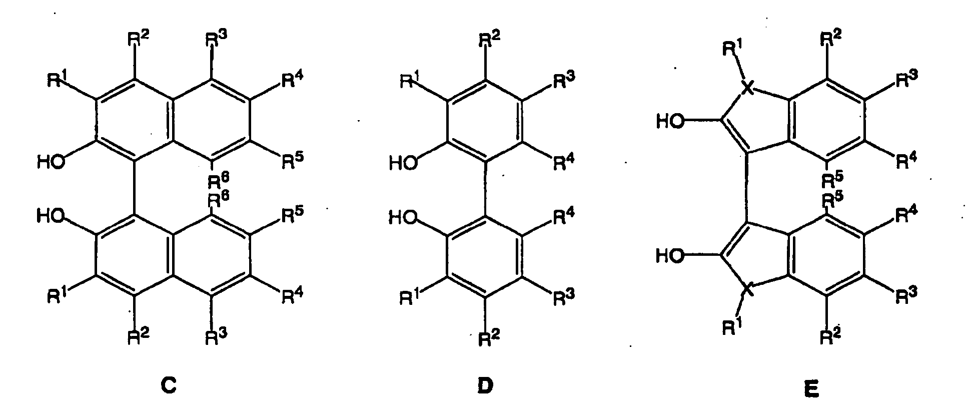

Im Fall der chiralen Hilfsmittel C besteht der sauerstoffhaltige Basisblock aus Binaphthol A mit den Resten R¹, R², R³, R⁴, R⁵ und R⁶, welche unabhängig voneinander die folgenden Gruppen sein können: Wasserstoff (H), gesättigte Kohlenwasserstoffe, ggf. funktionalisiert und/oder verbrückt (z.B. R¹ + R² = -(CH₂)₄-), aromatische oder heteroaromatische Gruppen, die funktionalisiert und/oder kondensiert sein können und ebenso cyclische Reste darstellen (beispielsweise R¹ + R² = ortho-Phenylen, entsprechend 4,4'-Dihydroxy-5,5'-bls(Phenanthryl), nicht-aromatische ungesättigte Kohlenwasserstoffe, wie Alkinylgruppen-C≡CR, welche ebenfalls funktionalisiert sein können, Silylgruppen, wie -SiMe₃, Halogen (-Cl, -Br, -F, -I), Nitro (-NO₂) oder Nitril (-CN) Gruppen oder Ester (-CO₂R), Amide (-C(O)NRR'), Amine (-NRR'), Ether (-OR), Sulfide (-SR) und Selenide (-SeR), worin R und R' Wasserstoff, gesättigte oder nicht-aromatische ungesättigte Kohlenwasserstoffe, die ggf. funktionalisiert sein können oder aromatische Reste, die ggf. funktionalisiert sein können, sind. Insbesondere umfasst die vorliegende Erfindung alle Kombinationen der in für R¹, R², R³, R⁴, R⁵ und R⁶ genannten Reste einschließlich aller C₁- oder C₂-symmetrischen Substitutionsmuster der Basisstruktur von Binaphthol. Ferner können eines oder mehrere Kohlenstoffatome des Binaphtholkems auch durch Heteroatome, wie Stickstoff, ersetzt werden. Bevorzugt stellt Bi-naphthol selbst (R' = R² = R³ = R⁴ = R⁵ = R⁶ = H) (A) den Basisblock dar, da es nicht nur im Bereich der asymmetrischen Katalyse eines der preiswertesten Hilfsmittel ist, aber auch weil eine hohe Effizienz erreicht wird, wenn mit diesem Diol hergestellte Diphosphonitliganden verwendet werden.

Im Fall des chiralen Diols D ist der Dihydroxy-Basisblock ein funktionalisiertes und hinsichtlich der Konfiguration stabiles Biphenol. Die Stabilität der Konfiguration Im Hinblick auf die axiale Chiralität ist gewährleistet, wenn R⁴ ≠ H (E. L. Eliel, S. H. Wilen, L. N. Mander, Stereochemistry of Organic Compounds, Wiley, New York, 1994). R¹ bis R⁴ zeigen die gleiche Bandbreite an Resten R¹ bis R⁶ aus der Verblndungsklasse C. Bevorzugt werden die besonders einfach zugänglichen Derivate D mit R¹ = R² = H und R⁴ = OCH₃ und R³ = Cl ausgewählt (D. J. Cram, R. C. Helgeson, S. C. Peacock, L. J. Kaplan, L. A. Domeier, P. Moreau, K. Koga, J. M. Mayer, Y. Chao, M. G. Siegel, D. H. Hoffman, G. D. Y. Sogah, J. Org. Chem. 1978, 43, 1930-1946).

Im Fall der chiralen Diole E ist der Dihydroxy-Basisblock ein funktionalisiertes, konfigurativ stabiles heteroaromatisches System, das sich von 2,2'-Dihydroxy-3,3'-bis(indolyl) (X = N), 2,2'-Dihydroxy-3,3'-bis(benzo[b]thiophenyl) (X = S) oder 2,2'-Dihydroxy-3,3'-bis(benzo[b]furanyl) (X = O) ableitet. Auch in diesen Fällen zeigen die Substituenten die gleiche Vielfalt wie In D. Substituent R¹ fehlt, wenn X = O oder X = S ist.

Chirale Spiro-Diole wie F (A.-G. Hu, Y. Fu, J.-H. Xie, H. Zhou, L.-X. Wang, Q.-L Zhou, Angew. Chem. Int. Ed. 2002, 41, 2348-2350), von Paracyclophan abgeleitete Diole G oder C1-oder C2-symmetrische Diole mit zentraler Chiralität, z.B. 1,3-Diole oder Diole vom Typ H, können auch als Komponenten bei der Synthese von Diphosphonit-Liganden eingesetzt werden.

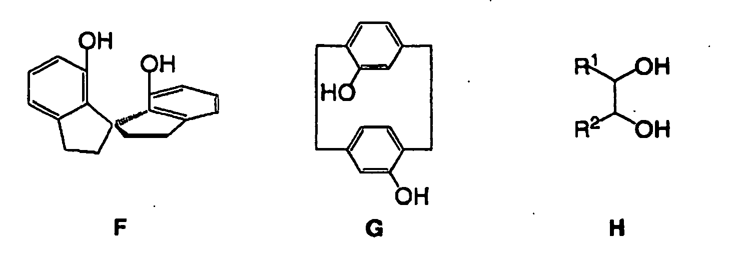

Die Reste R¹ und R² in den Diolen H können identisch (C₂-Symmetrie) oder unterschiedlich sein (C₁-Symmetrie). Sie können einen gesättigten Kohlenwasserstoff darstellen, der gegebenenfalls funktionalisiert sein kann, wie in den Fällen von 1,3-Dioleinheiten von geschützten Kohlenhydraten. Mögliche Reste schließen auch aromatische oder hetero-aromatische Gruppen ein, wie Phenyl, Naphthyl oder Pyridyl, welche selbst wieder funktionalisiert sein können, falls dieses erwünscht oder erforderlich ist. Es ist auch möglich, dass die Reste Ester- oder Amidgruppen aufweisen, wie -CO₂CH₃, -CO₂C₂H₅, -CO₂-i-C₃H₇ oder -CO[N(CH₃)₂], -CO[N(C₂H₅)₂] oder - CO[N(i-C₃H₇)₂], wobei die entsprechenden Diole H Weinsäure-Derivate sind.

Die bevorzugten Diphosphonit-Liganden bei der Ru-katalysierten Hydrierung von Ketonen, β-Ketoestern und Ketiminen sind solche, die sich von den Diolen **A, B** oder **D** ableiten (d.h. mit R¹ = R² = H; R³ = Cl; R⁴ = OCH₃).

Eine der wirksamsten und daher bevorzugten Liganden ist das Disphosponit **XI** oder dessen Analoga, in welchen der BINOL-Basisblock durch die chiralen Diole **B** oder **D** ersetzt worden Ist (z.B. R¹ = R² = H; R³ = Cl; R⁴ = OCH₃). Da jedoch kein Ligand allgemeingültig eingesetzt werden kann, müssen auch die anderen Diphosphonite berückslchtigt werden, wenn bestimmte Substrate hydriert werden sollen. Beispielsweise ist bei der Hydrierung von β- Ketoestern **III,** der Ligand X, der sich von Diphenylether ableitet, bevorzugt.

Die Erfindung setzt Metallkomplexe als Katalysatoren ein, die durch Umsetzung der oben definierten chiralen Diphosphonite mit Rutheniumsalzen, von denen eine große Vielzahl verfügbar sind (Encyclopedida of Inorganic Chemistry (R. B. King, Ed.), Vol. 7, Wiley, New York, 1994; Comprehensive Coordination Chemistry (G. Wilkinson, Ed.), Chapter 45, Pergamon Press, Oxford, 1987). Vorzugsweise werden Ru(II)-Salze verwendet, es können aber auch Ru(III)-Salze eingesetzt werden, die unter den Reaktionsbedingungen zu Ru(II) reduziert werden. Typische Beispiele schließen solche Verbindungen ein wie RuX₂ (X = Cl, Br, I, SC₆H₅, AcAc, OTf), aber auch **M, N, O, P** (worin X = Cl, Br, I, SPh, OPh, OAc, AcAc oder NHAc), **Q** (worin X = Cl, Br, I, SPh, OPh, OAc, AcAc oder NHAc), **R** (worin X = Cl, Br, I, SPh, OPh, OAc, AcAc oder NHAc), **S** oder **T.** Typische Ru(III)-Salze umfassen RuX₃ (X = Cl, Br, I, SPh, OPh, OAc, AcAc oder NHAc).

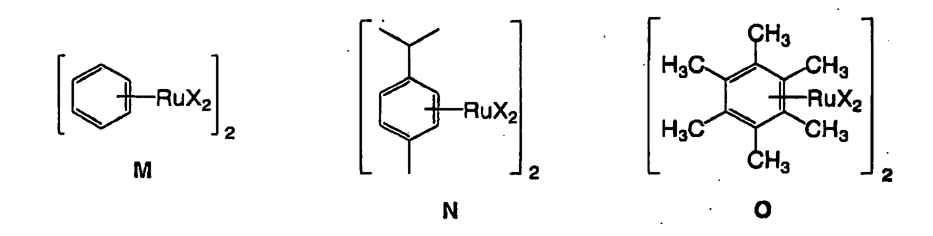

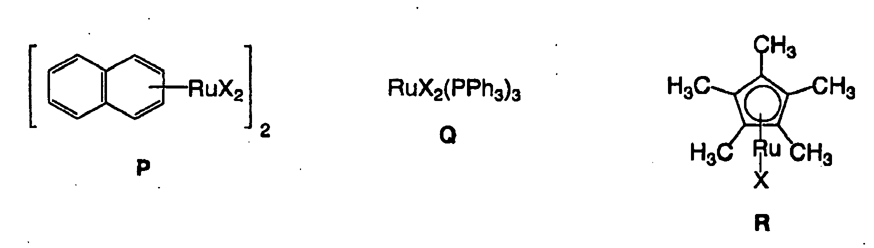

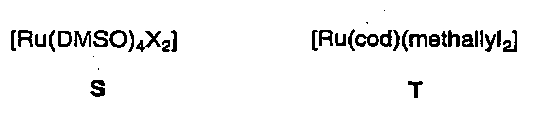

Diese Salze, von denen einige im Handel erhältlich sind, werden einfach mit den beschriebenen chiralen Diphosphoniten unter Bildung der Katalysatoren umgesetzt. Das Verhältnis von Diphosphonit zu Ru kann zwischen 2:1 und 4:1, vorzugsweise bei 2,5:1 liegen. Im allgemeinen bilden sich die bevorzugten Katalysatoren, wenn ein Verhältnis von 2:1 ausgewählt wird, aber ein Überschuss an Liganden kann in einigen Fällen von Vorteil sein. Einige der besten Katalysatoren für die Reduktion von Ketonen **I** werden gebildet, wenn die Salze der Vorstufe **M** oder **N** (X = Cl) mit dem Diphosphonit **XI** behandelt werden. Für den Fall, dass β-Ketoester **III** reduziert werden, werden die bevorzugten Katalysatoren durch die Behandlung der Salze **M** oder **N** mit dem Diphosphonit X gebildet.

Als Reduktionsmittel kann eine Vielzahl von Verbindungen eingesetzt werden, insbesondere bei der Hydrierung basierend auf der Verbindung von H₂ oder bei der Transfer-Hydrierung, bei welcher die Verwendung von Agentien wie Ameisensäure, Alkoholen, Natriumdithlonit oder NaH₂PO₂. Gemäß der vorliegenden Erfindung ist eine der am meisten bevorzugten Varianten die Transfer-Hydrierung unter Verwendung eines Alkohols sowohl als Reduktionsmittel als auch als Lösungsmittel. Eine große Vielzahl von Alkoholen sind dafür geeignet, wobei Isopropanol oder Cyclohexanol typischerweise eingesetzt werden. Isopropanol ist bevorzugt. In einigen Ausführungsformen der vorliegenden Erfindung wird die Hydrierung oder Transfer-Hydrierung in Gegenwart einer Base durchgeführt. Typische Basen sind NaOH, KOH, MgO, Na₂CO₃, K₂CO₃, NaF, KF, NaOCH(CH₃)₂, KOCH(CH₃)₂, NaOC(CH₃)₃ oder KOC(CH₃)₃, bevorzugte Basen NaOH, KOH, NaOC(CH₃)₃ oder KOC(CH₃)₃.

Typische Ketone, die der enantioselektiven Reduktion unter Verwendung der Katalysatoren und Verfahren der vorliegenden Erfindung gut zugänglich sind, sind die Ketone der Formeln **Ia - m.**

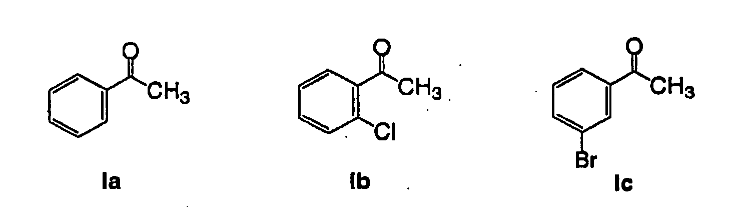

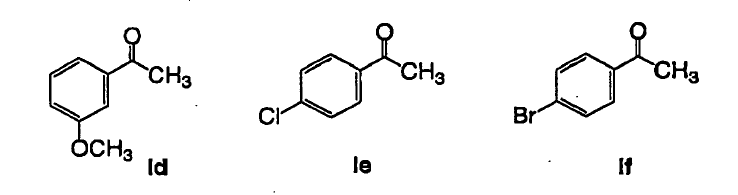

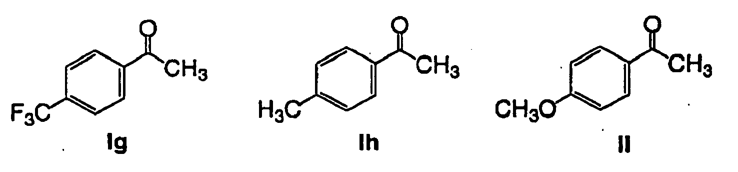

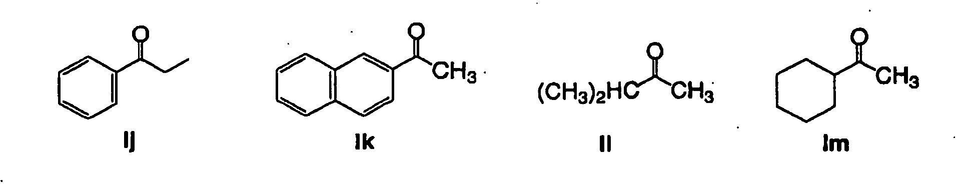

Typische β-Ketoester, die der asymmetrischen Ru-katalysierten Reduktion unterworfen werden, sind **IIIa** - **e,** aber R¹ und R² können entsprechend variiert werden, falls dies erforderlich ist.

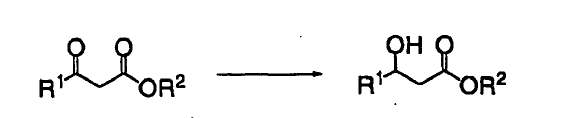

**IIIa** R¹ = R² = CH₃ **IV**

**b** R¹ = CH₃; R² = C₂H₅

**c** R¹ = C₂H₅; R² = CH₃

**d** R¹ = n-C₃H₇; R² = C₂H₅

**e** R¹ = C₆H₅; R² = C₂H₅

**f** R¹ = 2-thiophen-yl(C₄H₃S); R² = CH₃

Typische entsprechend Substrate sind solche β-Ketoester mit einem stereogenen Zentrum an der 2-Position wie **XXI** oder **XXIII,** welche ebenfalls reduziert werden können.

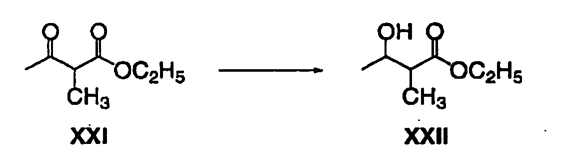

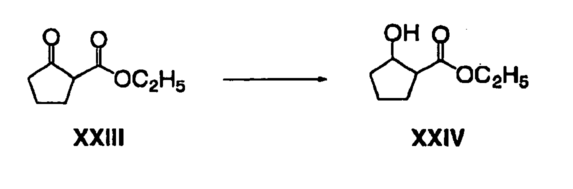

Typische prochirale Ketimine, die der Reduktion mit den Ru-Katalysatoren im erfindungsgemäßen Verfahren unterworfen werden, sind solche mit den Formeln **XXVa** - **b** oder **XXVII:**

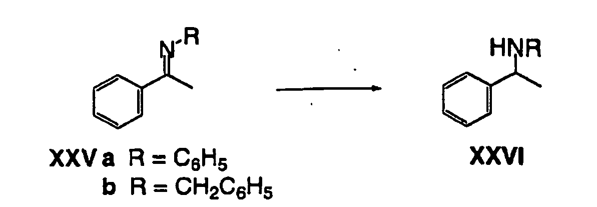

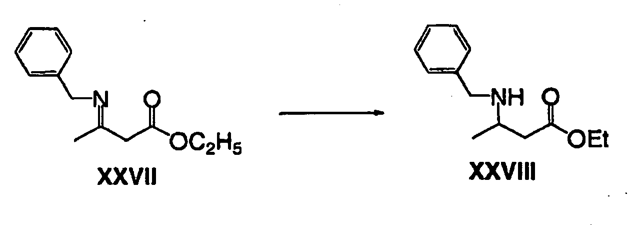

### Beispiele

### Typisches Verfahren für die asymmetrische Transfer-Hydrierung:

[RuCl₂(*p*-cymene]₂ (**N**) (1,22 mg, 2 µmol) und ein chiraler Diphosphonit-Ligand wie **XI** (0,010 mmol) wurden in trockenem Isopropanol (2,5 ml) bei 80°C 1 h unter Argon erhitzt. Nachdem das Gemisch auf Raumtemperatur abgekühlt war, wurde eine Base NaOH (0,04 mmol; 0,5 ml einer 0,08 M Lösung in Isopropanol) oder KOC(CH₃)₃ (0,04 mmol; 0,5 ml einer 0,08 M Lösung in Isopropanol) hinzugefügt, nachfolgend wurde ein Keton, wie Acetophenon (0,4 mmol) zugegeben. Das Reaktionsgemisch wurde bei 40°C unter Argon über einen definierten Zeitraum (üblicherweise 16-96 h) gerührt. Proben wurden der Reaktionslösung entnommen und über eine kleine Menge Silicagel vor der GC-Analyse zur Besstimmung der Umwandlungen und der ee-Werte durch Gaschromatographie gegeben.

### Typisches Verfahren für die asymmetrische H₂-Hydrierung:

[Ru(benzol)Cl₂]₂ (**N**) (16 mg, 0,032 mmol) und ein Diphosphonit (0,067 mmol) wurden in ein 25 ml Schlenkrohr gefüllt. Das Rohr wurde dreimal mit Argon gespült, bevor trockenes Dimethylformamid (DMF) (3 ml) zugegeben wurde. Das erhaltene Gemisch wurde auf 100°C 30 Minuten erwärmt und dann auf 60°C abgekühlt. Das Lösungsmittel wurde Im Vakuum entfernt, wobei der Katalysator als hellgrün-gelber Feststoff erhalten wurde. Dieser Katalysator wurde in trockenem Dichlormethan (8 ml) gelöst und gleichmäßig auf 8 Violen (jeweils 1 ml) verteilt, welche bereits dreimal mit Argon gespült worden waren. Ein Keton wie ein β-Ketoester (**III**) (0,8 mmol) wurde in jedes Gefäß gefüllt, anschließend wurden jeweils 3 ml Ethanol zugegeben. Diese wurden dann in einen Hochdruckautoklaven überführt. Nachdem dreimal mit H₂ gespült worden war, wurde der Autoklav mit H₂ auf 60 bar Druck gesetzt, die Reaktionen wurden bei 60°C über 20 h magnetisch gerührt. Der Autoklav wurde anschließend auf Raumtemperatur abgekühlt und H₂ wurde vorsichtig abgelassen. Proben wurden jeder Reaktionslösung entnommen und vor der GC-Analyse über eine kleine Menge Silicagel gegeben, um die Umwandlungen und ee-Werte zu bestimmen. Die absolute Konfiguration wurde im Vergleich mit bekannten in der Literatur beschriebenen Verbindungen bestimmt.

In Tabelle 1 werden die Ergebnisse, die durch die voranstehend beschriebenen Verfahren für die asymmetrische Transfer-Hydrierung von Ketonen, üblicherweise unter Verwendung des Diphosphonits **XI** als chiralen Liganden zusammengefasst.

**Tabelle 1. Typische Ergebnisse einer asymmetrischen Ru-katalysierten Transferhydrierung von β-Ketoestern unter Verwendung des gängigen Verfahrens (s. oben) und Diphosphonite XI als Liganden L* hergestellt mit (R)-BINOL; Bu^{t} = C(CH₃)₃.**

| Nr. | Keton | Base | L*/Ru | Zeit (h) | Umwandlung (%) | *ee* (%) | Konfiguration des Produktes |
|---|---|---|---|---|---|---|---|
| 1 | Ia | KOBu*^{t}* | 4 | 28 | 91 | 97 | *R* |
| 2 | Ia | NaOH | 2.5 | 20 | 88 | 97 | *R* |
| 3 | Ia | NaOH | 2.5 | 40 | 93 | 98 | *R* |
| 4 | Ib | KOBu*^{t}* | 4 | 30 | 50 | 98 | *R* |
| 5 | Ib | NaOH | 2.5 | 26 | 83 | 99 | *R* |
| 6 | Ib | NaOH | 2.5 | 40 | 90 | 99 | *R* |
| 7 | Ic | KOBu*^{t}* | 4 | 28 | 100 | 95 | *R* |
| 8 | Ic | NaOH | 2.5 | 16 | 100 | 96 | *R* |
| 9 | Id | NaOH | 2.5 | 40 | 63 | 93 | *R* |
| 10 | Id | NaOH | 2.5 | 96 | 91 | 93 | *R* |
| 11 | Ie | KOBu*^{t}* | 4 | 22 | 96 | 96 | *R* |
| 12 | Ie | NaOH | 2.5 | 16 | 98 | 95 | *R* |
| 13 | If | NaOH | 2.5 | 26 | 98 | 95 | *R* |
| 14 | Ig | NaOH | 2.5 | 16 | 100 | 97 | *R* |
| 15 | Ih | KOBu*^{t}* | 4 | 30 | 67 | 95 | *R* |
| 16 | Ih | NaOH | 2.5 | 26 | 65 | 94 | *R* |
| 17 | Il | KOBu*^{t}* | 4 | 30 | 50 | 89 | *R* |
| 18 | Ij | NaOH | 2.5 | 26 | 65 | 93 | *R* |
| 19 | Ik | NaOH | 2.5 | 22 | 56 | 93 | *R* |
| 20 | Il | NaOH | 2.5 | 26 | 98 | 98 | *S* |
| 21 | Im | NaOH | 2.5 | 26 | 96 | 99 | *R* |

Die Ergebnisse einer asymmetrischen H₂-Hydrierung von β-Ketoestern **III** sind in Tabelle 2 zusammengefasst.

**Tabelle 2. Ergebnisse einer asymmetrischen Ru-katalysierten H₂-Reduktion von β-Ketoestern unter Verwendung des gängigen Verfahrens (s. oben) und Diphosphonite X als Ligand hergestellt mit (S)-BINOL.**

| β-Ketoester | Umwandlung (%) | *ee* (%) | Konfiguration des Produktes |
|---|---|---|---|
| **IIIa** | 100 | 93 | *S* |
| **IIIb** | 100 | 95 | *S* |
| **IIIc** | 100 | 95 | *S* |
| **IIId** | 100 | 97 | *S* |
| **IIIe** | 100 | 95 | *R* |
| **XXI** | 100 | 95/95^{a)} | anti-diastereomer: (2*S*,3*S*) |
| **XXIII** | 100 | 99^{b)} | anti-diastereomer: (1*S*,2*S*) |

| | | | |
|---|---|---|---|
| a) Diastereomerenverhältnis ist 1:1, jeweils 95% ee; b) nur ein Diastereomer (96:4). | | | |

## Patentansprüche

1. Verfahren zur asymmetrischen Ruthenium-katalysierten Reduktion von prochiralen Ketonen, β-Ketoestern und Ketiminen, worin chirale Rutheniumkomplexe hergestellt durch die Reaktion eines Rutheniumsalzes mit einem chiralen Diphosphonit, in welchem das Diphosphonit abgeleitet ist aus einem chiralen Diol mit der allgemeinen Struktur, gezeigt in Schema 1, verwendet werden 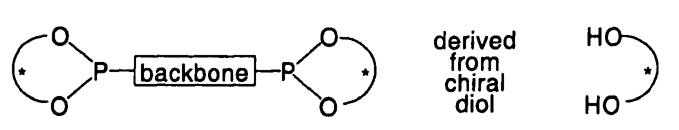 Scheme 1. Chiral phosphonites derived from chiral diols
das **dadurch gekennzeichnet ist, dass** Rückgrat achiral ist und sich von einem der Reste des **U1** - **U15** ableitet

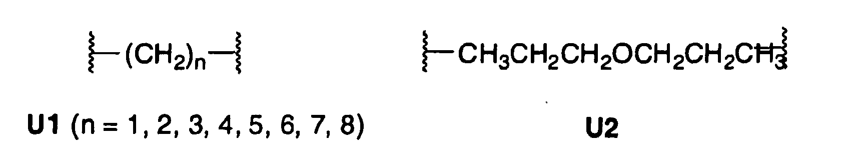

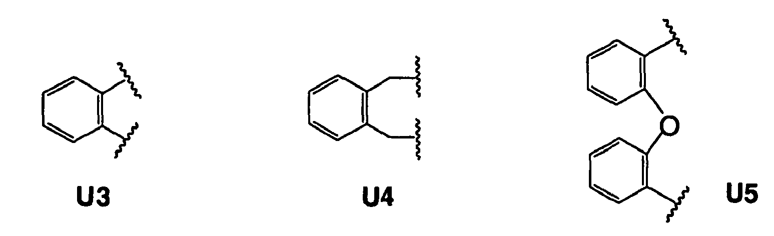

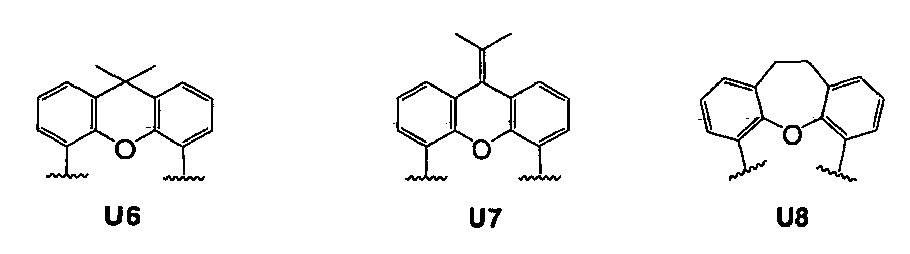

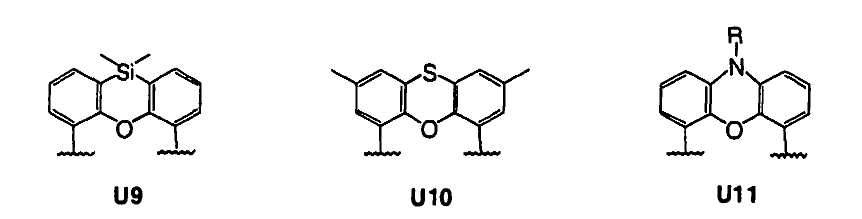

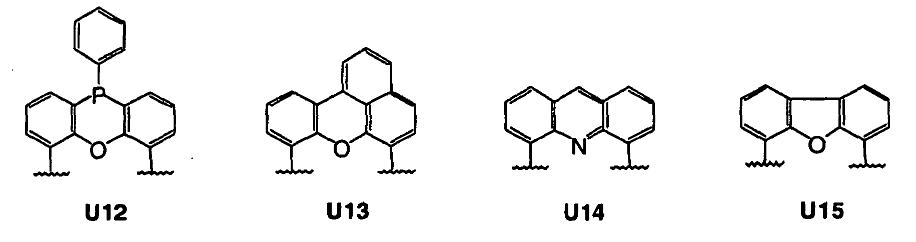

2. Verfahren nach Anspruch 1, in welchem das bei der Synthese der Liganden verwendete Diol axiale Chiralität aufweist, wie in **C, D** oder **E** mit den Gruppen R¹, R², R³, R⁴, R⁵ und R⁶, die jeweils unabhängig voneinander bedeuten: Wasserstoff, gesättigte Kohlenstoffketten, die jeweils funkionalisiert und/oder verbrückt sein können, aromatische oder hetero-aromatische Reste, die jeweils funktionalisiert und/oder verbrückt sein können, nicht aromatische ungesättigte Kohlenstoffketten, die jeweils funktionalisiert sein können, Silylgruppen, Halogen (F, Cl, Br oder J), Nitro-, Nitril-, Ester-, Amid-, Amin-, Ether- oder Thioether-Reste.

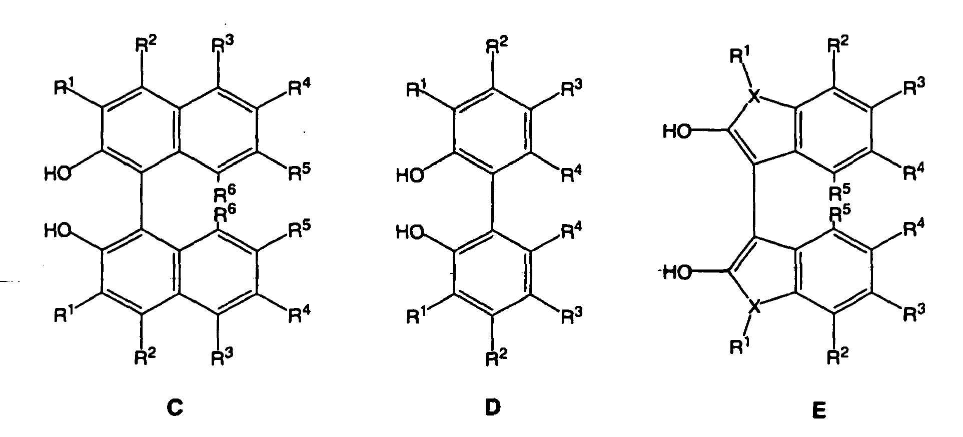

3. Verfahren nach Anspruch 1 oder 2, worin das an der Synthese der Ruthenium-Komplexe verwendete Diol mit axialer Chiralität der Ligand **A, B** oder **D1** ist.

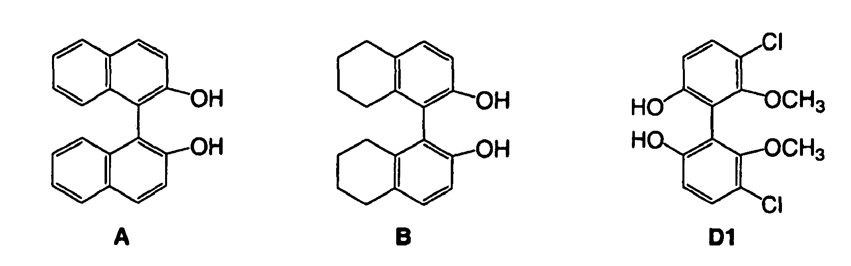

4. Verfahren nach einem der Ansprüche 1 bis 3, worin das bei der Synthese der Diphosphonite verwendete chirale Diol ein Spiro-Diol ist, wie **F** oder ein Diol mit zentraler Chiralität, wie **G** oder **H**, in welchen R¹ = R² oder R¹ ≠ R², wobei diese Reste Methyl, Ethyl, Propyl, Butyl, Phenyl, Naphthyl, Carboxy oder Carboxamido sind.

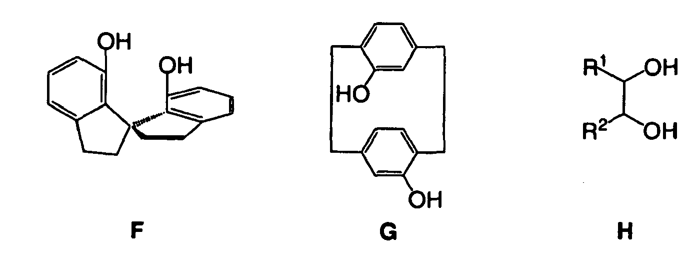

5. Verfahren nach einem der Ansprüche 1 bis 4, worin zur Herstellung der Ruthenium-Komplexe Ru(II)-Salze verwendet werden.

6. Verfahren nach Anspruch 5, worin die Ru(II)-Salze **M, N, O, P, Q, R, S** oder **T** sind: 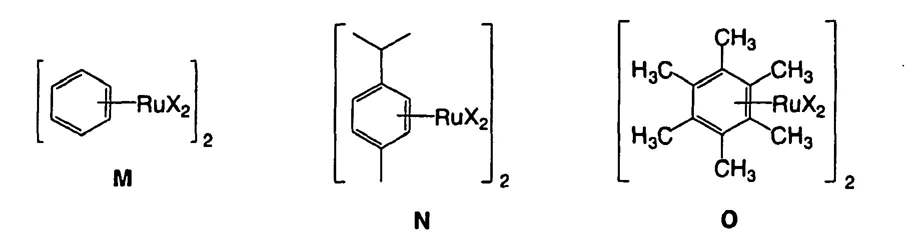 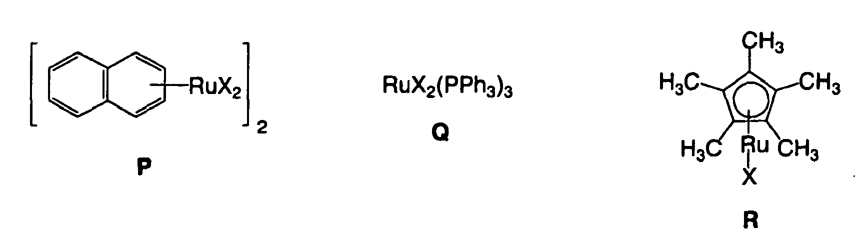 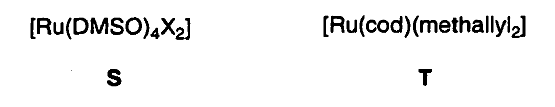 in denen X = Cl, Br, I, OAc, OC₆H₅, SC₆H₅, AcAc, OTf, NHAc.

7. Verfahren nach einem der Ansprüche 1 bis 6, worin Ru(III)-Salze zur Herstellung der Ruthenium-Komplexe verwendet werden.

8. Verfahren nach Anspruch 7, worin das Ru(III)-Salz RuX₃ (X = Cl, Br, I, SC₆H₅, AcAc, OTf) ist.

9. Verfahren nach einem der Ansprüche 1 bis 8, worin H₂ als Reduktionsmittel verwendet wird.

10. Verfahren nach einem der Ansprüche 1 bis 8, in welchem ein Alkohol, Ameisensäure, Natrium- oder Ammoniumformiat oder anorganische Reduktionsmittel vom Typ Na₂S₂O₄ oder NaH₂PO₂ in der Transfer-Hydrierung verwendet werden.

11. Verfahren nach Anspruch 10, in welchem Isopropanol oder Cyclohexanol als Reduktionsmittel verwendet wird.

12. Verfahren nach einem der Ansprüche 1 bis 11, in welchem eine Base zum Reaktionsgemisch hinzugeführt wird.

13. Verfahren nach Anspruch 12, in welchem die Base NaOH, KOH, MgO, Na₂CO₃, K₂CO₃, NaF, KF, NaOCH(CH₃)₂, KOCH(CH₃)₂, NaOC(CH₃)₃ oder KOC(CH₃)₃ ist.

## Claims

1. Process for asymmetric ruthenium-catalysed reduction of prochiral ketones, β-keto esters and ketimines, in which chiral ruthenium complexes, in which the diphosphonite is derived from a chiral diol having the general structure shown in scheme 1, are used 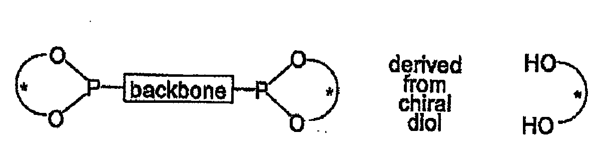 Scheme 1. Chiral phosphonites derived from chiral diols **characterized in that** the backbone is achiral and derives from one of the **U1-U15** radicals.

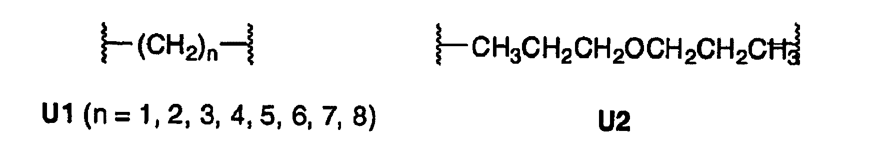

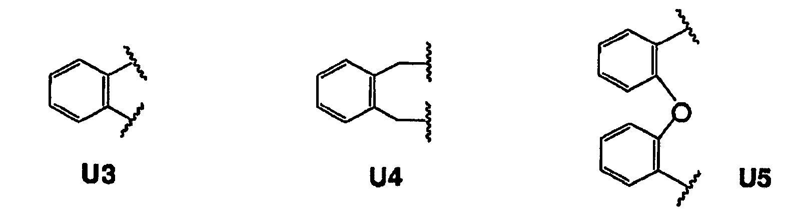

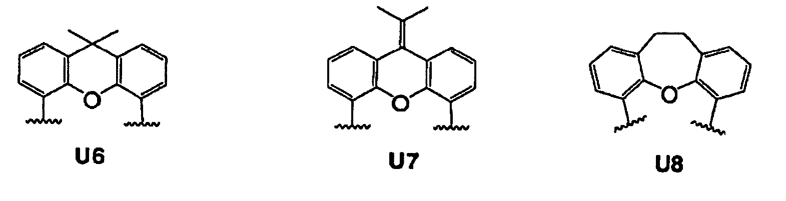

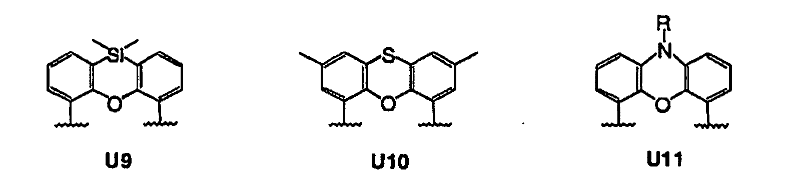

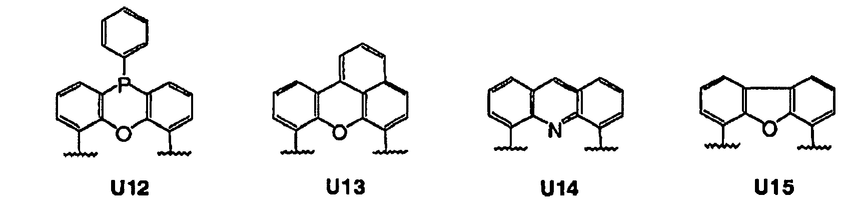

2. Process according to claim 1, in which the diol used in the synthesis of the ligands has axial chirality such as in **C, D** or **E** with R¹, R², R³, R⁴, R⁵ and R⁶ groups, each of which is independently: hydrogen, saturated carbon chains which may each be functionalized and/or bridged, aromatic or heteroaromatic radicals which may each be functionalized and/or bridged, nonaromatic unsaturated carbon chains which may each be functionalized, silyl groups, halogen (F, Cl, Br or I), nitro, nitrile, ester, amide, amine, ether or thioether radicals.

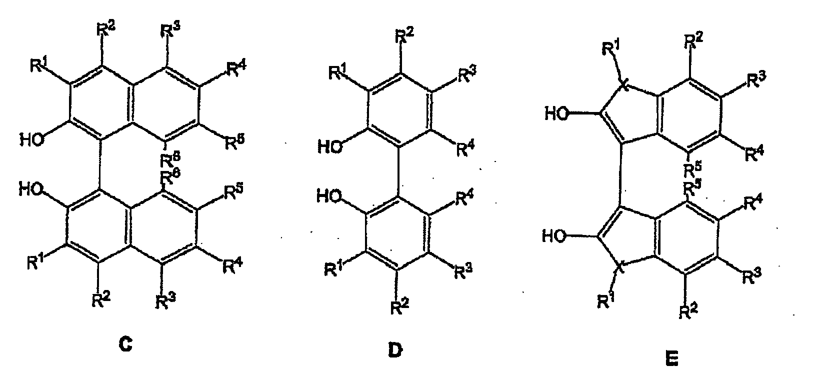

3. Process according to claim 1 or 2, in which the diol having axial chirality used in the synthesis of the ruthenium complexes is the ligand **A**, **B** or **D1.**

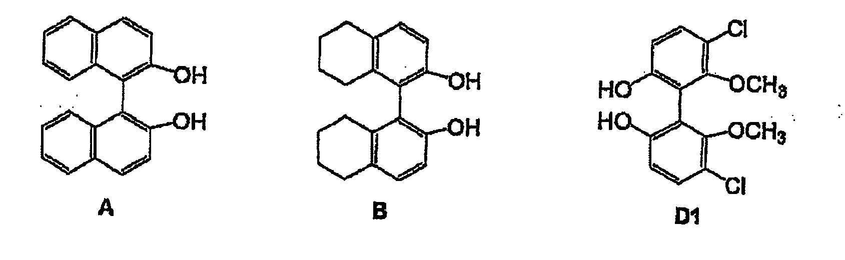

4. Process according to one of claims 1 to 3, in which the chiral diol used in the synthesis of the diphosphonites is a spiro-diol such as **F** or a diol having central chirality such as **G** or **H**, in which R¹ = R² or R¹ ≠ R², where these radicals are methyl, ethyl, propyl, butyl, phenyl, naphthyl, carboxyl or carboxamido.

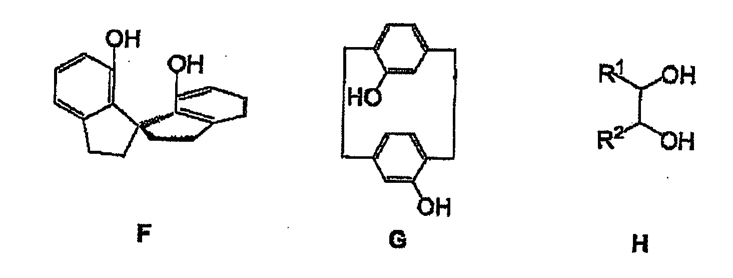

5. Process according to one of claims 1 to 4, in which the ruthenium complexes are prepared using Ru(II) salts.

6. Process according to claim 5, in which the Ru(II) salts are **M**, **N**, **O, P**, **Q, R**, **S** or **T**: 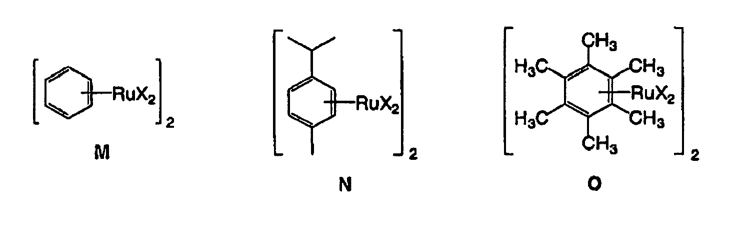 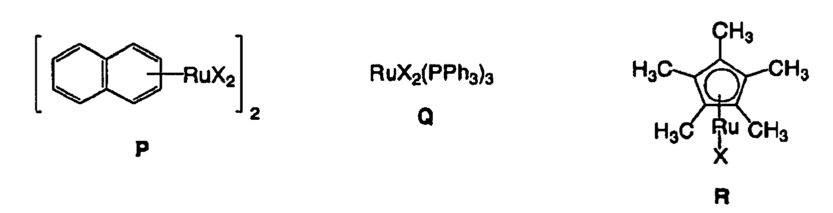 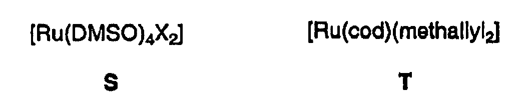 in which X = Cl, Br, I, OAc, OC₆H₅, SC₆H₅, AcAc, OTf, NHAc.

7. Process according to one of claims 1 to 6, in which Ru(III) salts are used for the preparation of the ruthenium complexes.

8. Process according to claim 7, in which the Ru(III) salt is RuX₃ (X = Cl, Br, I, SC₆H₅, AcAc, OTf).

9. Process according to one of claims 1 to 8, in which H₂ is used as a reducing agent.

10. Process according to one of claims 1 to 8, in which an alcohol, formic acid, sodium formate or ammonium formate or inorganic reducing agents of the Na₂S₂O₄ or NaH₂PO₂ type is used in the transfer hydrogenation.

11. Process according to claim 10, in which isopropanol or cyclohexanol is used as a reducing agent.

12. Process according to one of claims 1 to 11 in which a base is added to the reaction mixture.

13. Process according to claim 12, in which the base is NaOH, KOH, MgO, Na₂CO₃, K₂CO₃, NaF, KF, NaOCH(CH₃)₂, KOCH(CH₃)₂, NaOC(CH₃)₃ or KOC(CH₃)₃.

## Revendications

1. Procédé pour la réduction asymétrique, catalysée par du ruthénium, de cétones prochirales, de β-cétoesters prochiraux et de cétimines prochirales, dans lequel sont utilisés des composés complexes chiraux du ruthénium qui sont préparés par la réaction d'un sel de ruthénium avec une diphosphonite chirale, dans laquelle la diphosphonite est dérivée d'un diol chiral présentant la structure générale indiquée dans le schéma 1 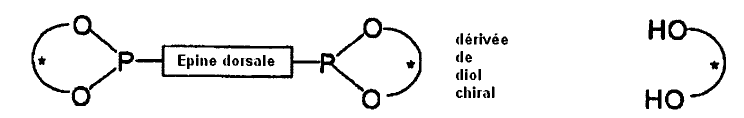 Schéma 1. Phosphonites chiraux dérivés de diols chiraux
**caractérisé en ce que** l'épine dorsale est achirale et est dérivée d'un des radicaux U1-U15

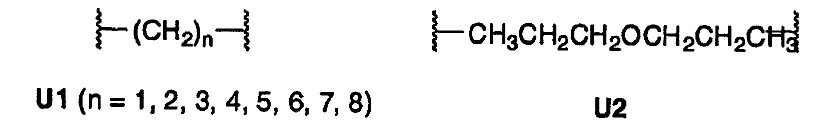

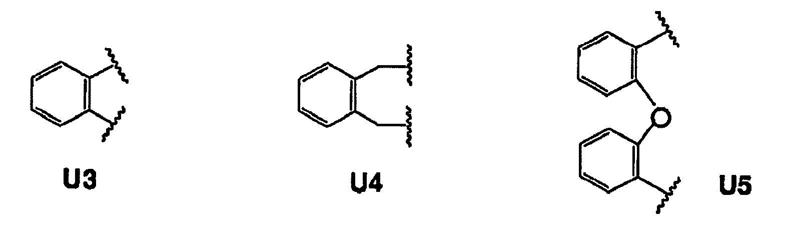

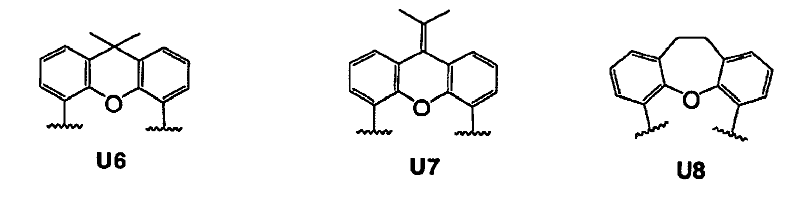

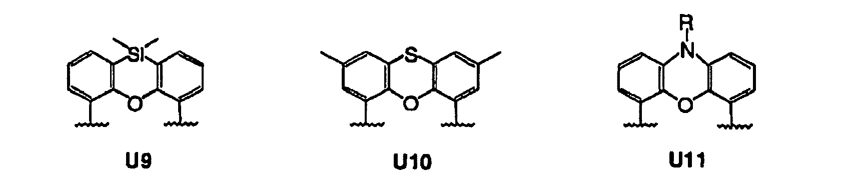

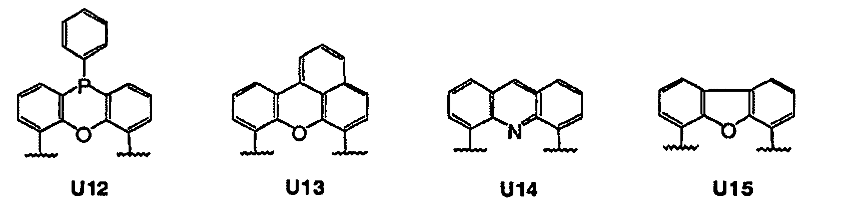

2. Procédé selon la revendication 1, dans lequel le diol utilisé lors de la synthèse des ligands présente une chiralité axiale, comme dans C, D ou E avec les groupes R¹, R², R³, R⁴, R⁵ et R⁶, qui signifient à chaque fois indépendamment l'un de l'autre : hydrogène, des chaînes carbonées saturées, qui peuvent à chaque fois être fonctionnalisées et/ou pontées, des radicaux aromatiques ou hétéroaromatiques, qui peuvent à chaque fois être fonctionnalisés et/ou pontés, des chaînes carbonées insaturées non aromatiques, qui peuvent à chaque fois être fonctionnalisées, des groupes silyle, halogène (F, Cl, Br ou I), des radicaux nitro, nitrile, ester, amide, amine, éther ou thioéther.

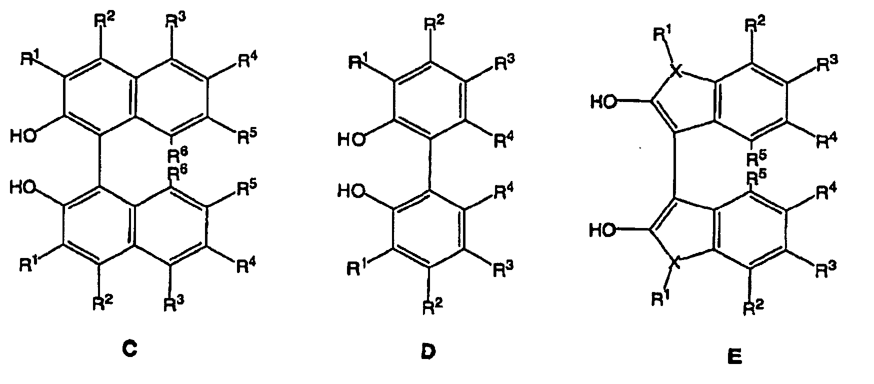

3. Procédé selon la revendication 1 ou 2, dans lequel le diol utilisé dans la synthèse des complexes de ruthénium avec une chiralité axiale est le ligand A, B ou D1.

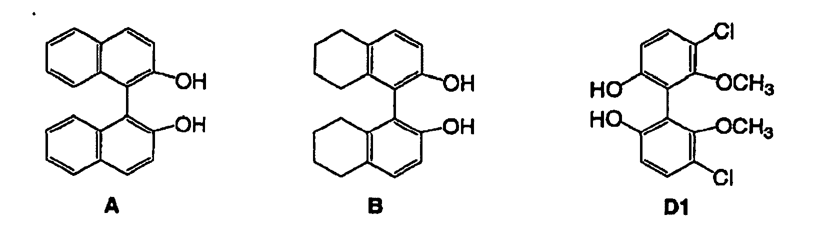

4. Procédé selon l'une quelconque des revendications 1 à 3, dans lequel le diol chiral utilisé lors de la synthèse des diphosphonites est un spiro-diol, tel que F ou un diol avec une chiralité centrale, tel que G ou H, dans lequel R¹ = R² ou R¹ ≠ R², ces radicaux représentant méthyle, éthyle, propyle, butyle, phényle, naphtyle, carboxy ou carboxamido.

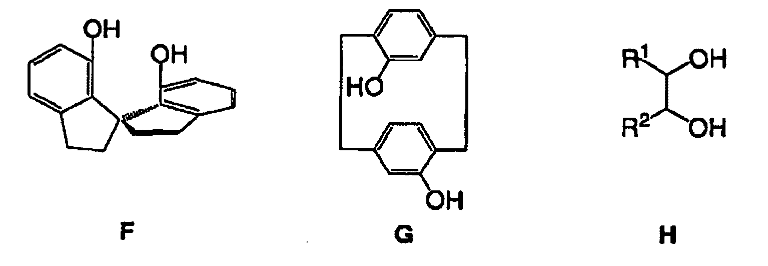

5. Procédé selon l'une quelconque des revendications 1 à 4, dans lequel, pour la préparation des complexes de ruthénium, on utilise des sels de Ru (II).

6. Procédé selon la revendication 5, dans lequel les sels de Ru (II) sont M, N, O, P, Q, R, S ou T : 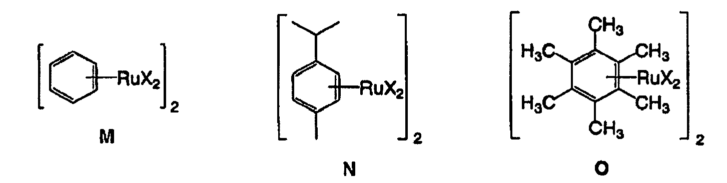 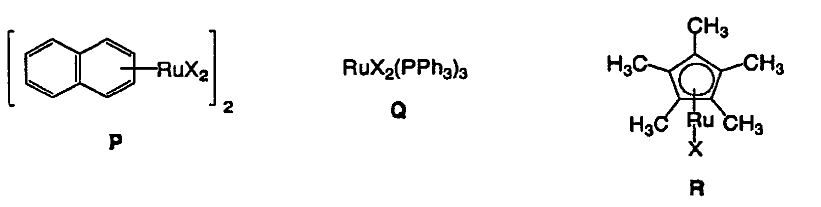 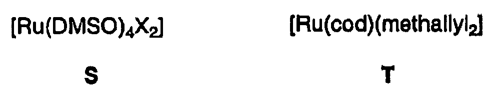 dans lesquels X = Cl, Br, I, OAc, OC₆H₅, SC₆H₅, AcAc, OTf, NHAc.

7. Procédé selon l'une quelconque des revendications 1 à 6, dans lequel on utilise des sels de Ru (III) pour la préparation des complexes de ruthénium.

8. Procédé selon la revendication 7, dans lequel le sel de Ru (III) est RuX₃ (X = Cl, Br, I, SC₆H₅, AcAc, OTf).

9. Procédé selon l'une quelconque des revendications 1 à 8, dans lequel on utilise H₂ comme réducteur.

10. Procédé selon l'une quelconque des revendications 1 à 8, dans lequel on utilise un alcool, de l'acide formique, du formiate de sodium ou d'ammonium ou des réducteurs inorganiques du type Na₂S₂O₄ ou NaH₂PO₂ dans l'hydrogénation de transfert.

11. Procédé selon la revendication 10, dans lequel on utilise de l'isopropanol ou du cyclohexanol comme réducteur.

12. Procédé selon l'une quelconque des revendications 1 à 11, dans lequel une base est introduite dans le mélange réactionnel.

13. Procédé selon la revendication 12, dans lequel la base est NaOH, KOH, MgO, Na₂CO₃, K₂CO₃, NaF, KF, NaOCH(CH₃)₂, KOCH(CH₃)₂, NaOC(CH₃)₃ ou KOC(CH₃)₃.
